# EUROPEAN PATENT SPECIFICATION

(11) **EP 1 518 582 B1**
(45) Date of publication and mention of the grant of the patent: **11.06.2008**
(21) Application number: 04255818.9
(22) Date of filing: 24.09.2004
(51) Int. Cl.: A61M 25/00

(54) **Rapid-exchange balloon catheter with hypotube shaft**
Schnellwechsel-Ballonkatheter mit zentralem Schaft
Cathéter a échange rapide avec tube hypodermique

(30) Priority: 29.09.2003 US 673650
(43) Date of publication of application: 30.03.2005
(73) Proprietor: Cordis Corporation, Miami Lakes, Florida 33014 (US)
(72) Inventor: Van Erp, Wilhelmus Petrus Martinus Maria, 9351 KS Leek (NL); Schulting, Edwin A., 9751 VD Haren (NL); Stapelbroek, Martinus B., 9451 AH Rolde (NL)
(74) Representative: Belcher, Simon James

(56) References cited:
- DE-A1- 4 200 030
- US-A- 5 370 615
- US-A1- 2001 031 979
- US-A1- 2003 078 537

## Description

The present invention relates generally to medical devices, and more particularly to a balloon catheter with a hypotube shaft.

Balloon catheters are used in a variety of therapeutic applications, including intravascular catheters for procedures such as angioplasty. Approximately one million angioplasties are performed worldwide each year to treat vascular disease, including coronary, neurological and peripheral blood vessels partially or totally blocked or narrowed by a lesion or stenosis. By way of example, the present invention will be described in relation to coronary and peripheral angioplasty treatments. However, it should be understood that the present invention relates to any balloon catheter having a hypotube shaft according to the present invention as recited in the following claims, and is not limited to angioplasty.
Most balloon catheters have a relatively long and flexible tubular shaft defining one or more passages or lumens, and have an inflatable balloon attached near one end of the shaft. This end of the catheter where the balloon is located is customarily referred to as the "distal" end, while the other end is called the "proximal" end. The proximal end of the shaft generally leads to a hub coupling at the proximal end for connecting the lumen(s) to various equipment.

The interior of the balloon is connected to one of the lumen(s) extending through the shaft for the purpose of selectively inflating and deflating the balloon. This lumen is of course referred to as the inflation lumen.

The catheter shaft also may define a second passage for slidingly receiving a guidewire, referred to as a guidewire lumen. The guidewire lumen extends between a distal guidewire port at the catheter distal end, and a proximal guidewire port. The catheter of the present invention has a "rapid exchange" configuration in which the proximal guidewire port is located somewhere between the balloon and the proximal hub. Structurally, the balloon may define an inflatable central portion defining an inflated size, flanked by a pair of proximal and distal conical portions, flanked by a pair of proximal and distal legs or collars. The proximal and distal collars may be affixed to the shaft.

Examples of this type of balloon catheter are shown in the following patents, which are co-owned with the present invention: United States Patent number 5,304,197, entitled "Balloons For Medical Devices And Fabrication Thereof," issued to Pinchuk et al. on April 19, 1994, and also in United States Patent number 5,370,615 (closest prior art), entitled "Balloon Catheter For Angioplasty," issued to Johnson on December 6,1994.

Common treatment methods for using such a balloon catheter include advancing a guidewire into the body of a patient, by directing the guidewire distal end percutaneously through an incision and along a body passage until it is located within or beyond the desired site. The term "desired site" refers to the location in the patient's body currently selected for treatment by a health care professional. The guidewire may be advanced before, or simultaneously with, a balloon catheter. When the guidewire is within the balloon catheter guidewire lumen, the balloon catheter may be advanced or withdrawn along a path defined by the guidewire. After the balloon is disposed within the desired site, it can be selectively inflated to press outward on the body passage at relatively high pressure to a relatively constant diameter, in the case of an inelastic or non-compliant balloon material.

This outward pressing of a constriction or narrowing at the desired site in a body passage is intended to partially or completely re-open or dilate that body passageway or lumen, increasing its inner diameter or cross-sectional area. In the case of a blood vessel, this procedure is referred to as angioplasty. The objective of this procedure is to increase the inner diameter or cross-sectional area of the vessel passage or lumen through which blood flows, to encourage greater blood flow through the newly expanded vessel. The narrowing of the body passageway lumen is called a lesion or stenosis, and may be formed of hard plaque or viscous thrombus.

Some balloon catheters are used to deliver and deploy stents or other medical devices, in a manner generally known in the art. Stents, for example, are generally tubular scaffolds for holding a vessel or body passage open.

It is desirable to provide a balloon catheter having an optimum combination of various performance characteristics, which may be selected among: flexibility, lubricity, pushability, trackability, crossability, low profile and others. Flexibility may relate to bending stiffness of a medical device (balloon catheter and/or stent, for example) in a particular region or over its entire length, or may relate to the material hardness of the components. Lubricity may refer to reducing friction by using low-friction materials or coatings. Pushability may relate to the column strength of a device or system along a selected path. Trackability may refer to a capability of a device to successfully follow a desired path, for example without prolapse. Crossability may be clarified by understanding that physicians prefer to reach the desired site with the balloon catheter while encountering little or no friction or resistance. Profile may refer to a maximum lateral dimension of the balloon catheter, at any point along its length.

The hypotube shaft of the present invention provides various advantages, which may include: pushability, torsional strength, low profile, etc. Some embodiments of the present invention may also provide additional benefits, including lower cost and ease of manufacture, a relatively few number of component parts, etc.

The balloon catheter has a shaft extending from a proximal end of the catheter to a distal end. The shaft has a proximal section and a distal polymer section. The proximal section includes a metal hypotube, which has a proximal cylindrical tubular portion, an intermediate tubular portion with a longitudinal indentation, and a distal portion. The longitudinal indentation may be shallower in a proximal direction, and deeper in the distal direction. The distal portion of the hypotube may have an arcuate cross-section or a wirelike shape, etc.

A proximal hub may be provided at the proximal end, affixed to the proximal hypotube. The hub has a locking fitting for connecting the inflation lumen to a device for inflation and deflation.

The distal section of the shaft includes an inner and outer tube extending generally between the hypotube and the balloon. The inner and outer tubes are affixed near their proximal ends a point on the hypotube; and they are affixed near their distal ends to the balloon. The inner tube defines a guidewire lumen extending from a distal guidewire port which is at or near the catheter distal end, to a proximal guidewire port which is at or near the proximal end of the inner tube. An inflation lumen is defined by the shaft, extending from a proximal inflation port defined by the proximal hub, through the hypotube and then through an annular space defined between the inner and outer tubes, into the balloon.

The hypotube defines a transition point at a distal end of the indented tubing portion, and at a proximal end of the distal portion. At or near this transition, the proximal ends of the inner and outer tubular bodies are sealed to the hypotube. At this transition seal, the proximal end of the inner tubular body may be partially received within a distal portion of the longitudinal indentation of the hypotube, and the outer tubular body proximal end surrounds and is sealed to both the inner body and the hypotube. A smooth flexibility transition is thus provided between the tubular portions of the hypotube and the more flexible distal shaft section.

In contrast to the distal shaft portion, the proximal hypotube portion of the shaft may have much greater column strength, which will tend to enhance the pushability of the balloon catheter, yet without adversely affecting flexibility in the distal portion of the shaft where flexibility is relatively more important.

Embodiments of the invention will now be described by way of example with reference to the accompanying drawings, in which:
Figure 1 is an external perspective view of a rapid-exchange balloon catheter;

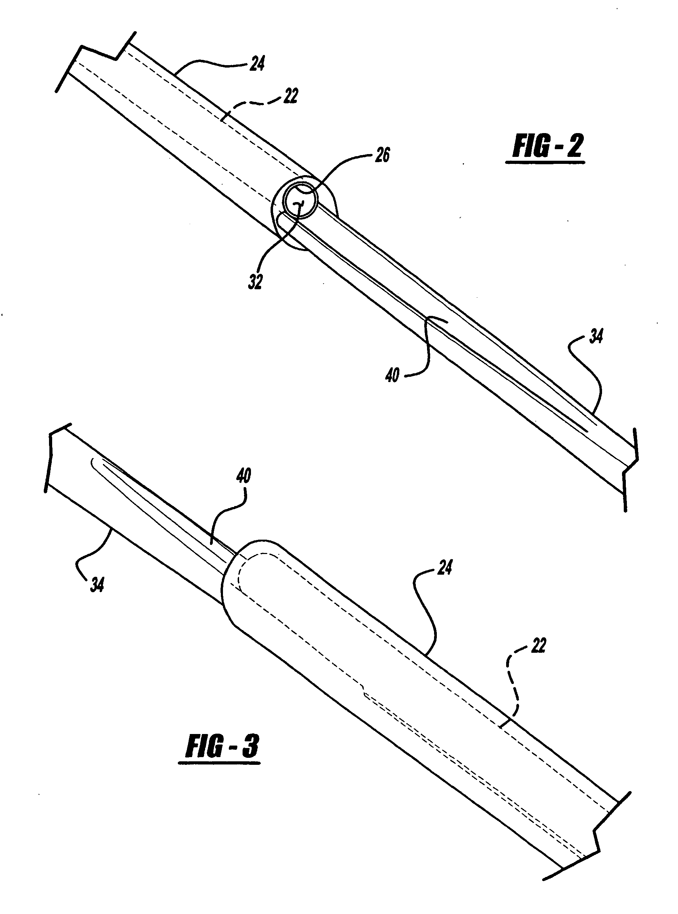

Figure 2 is a partial perspective view of an intermediate portion of a balloon catheter;
Figure 3 is a partial external perspective view of the intermediate portion of Figure 2;
Figure 4 is a partial external perspective view of a hypotube component;
Figure 5 is a cross-section view of an intermediate portion of a balloon catheter;
Figure 6 is a partial external perspective view of a proximal hub, strain relief, and hypotube;
Figure 7 is a side view of a hypotube;
Figure 8 is a top view of the hypotube of Figure 7;

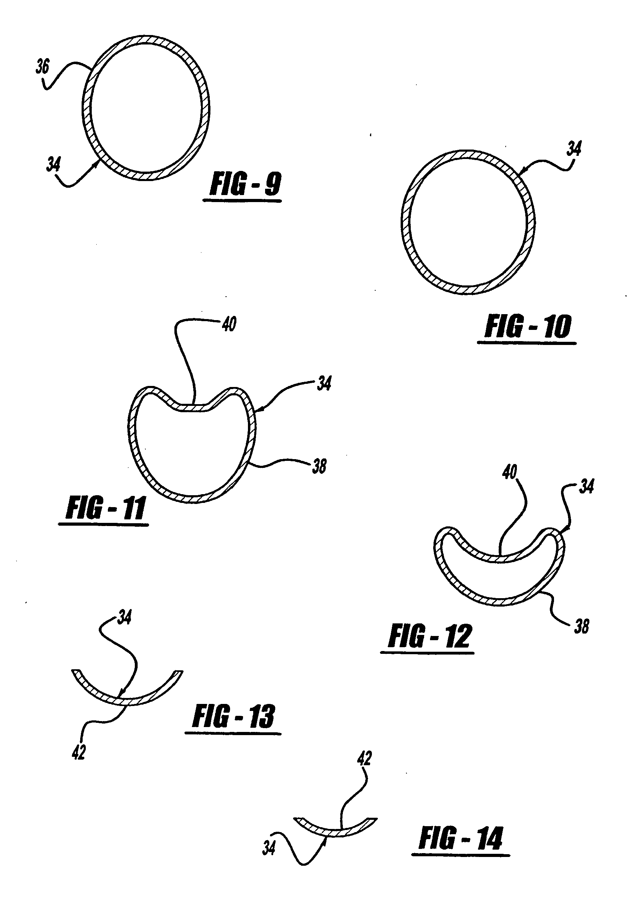

Figure 9-14 are cross-section views of the hypotube of Figures 7 and 8, along the lines 9-9 through 14-14, respectively;
Figure 15 is a side view of another hypotube;
Figures 16-20 are cross-section views of the hypotube of Figure 15, along the lines 16-16 through 20-20, respectively;

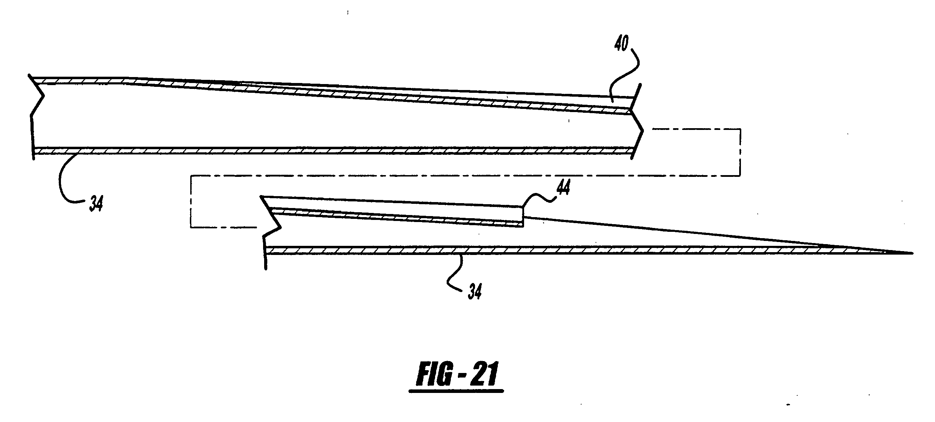

Figure 21 is a partial longitudinal cross-section view of a hypotube;
Figure 22 is a partial longitudinal cross-section view of a balloon catheter and stent; and

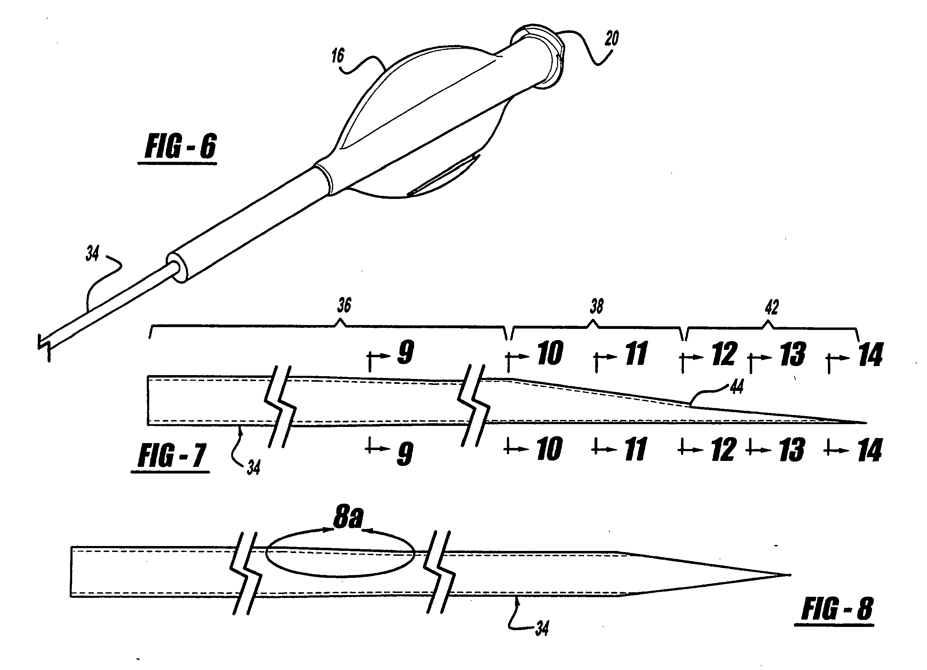

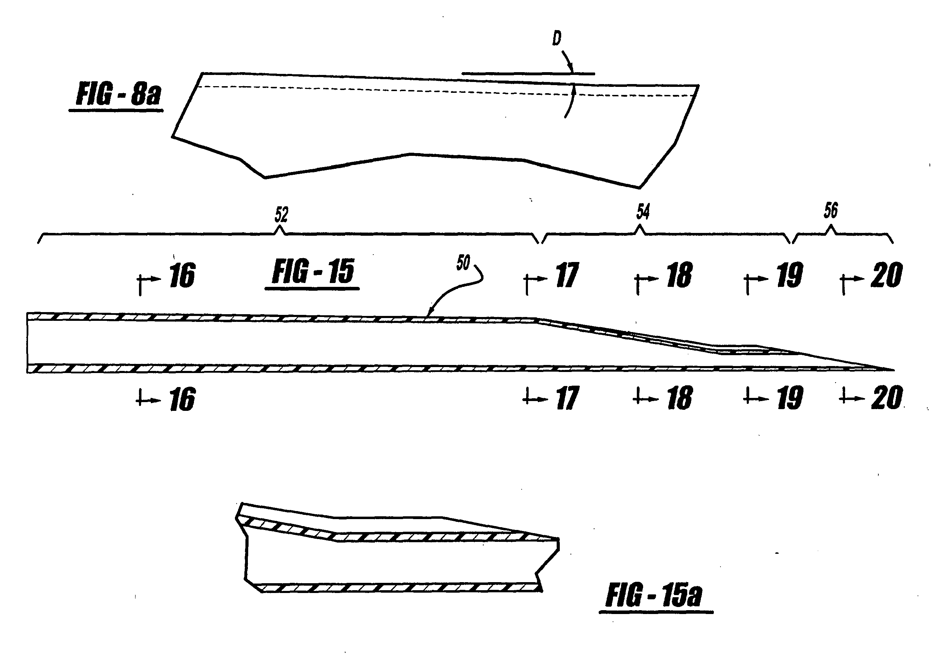

Figures 8a and 15a are partial expanded views of portions of hypotubes.

The following description of the preferred embodiments of the present invention is merely illustrative in nature, and as such it does not limit in any way the present invention, its application, or uses.

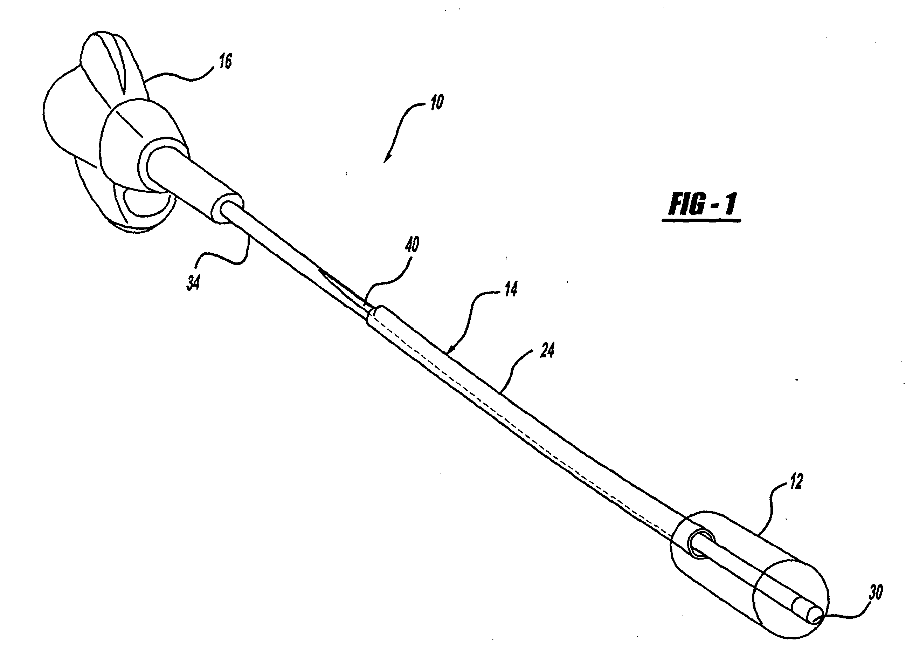

Referring to the drawings, a balloon catheter system is depicted, with one of the preferred embodiments of the present invention being shown generally at 10. The balloon catheter of Figure 1 has an inflatable balloon 12, a relatively long and flexible tubular shaft 14, and a hub 16. The balloon 12 is affixed to the shaft 14 near a distal end of the shaft 14, and the hub 16 is affixed to the proximal end of the shaft 14.

The shaft defines at least two passages or lumens, one ofwhich is an inflation lumen 18 connected to the balloon 12 for the purpose of selectively inflating and deflating the balloon 12. The inflation lumen 18 thus provides fluid communication between the interior of the balloon 12 at the distal end of the inflation lumen, and a hub inflation port 20 having a coupling or luer-lock fitting at the proximal end for connecting the inflation lumen to a source of pressurized inflation fluid (not shown) in the conventional manner.
A second lumen defined by the catheter 10 is a guidewire lumen 26 is adapted to receive an elongated flexible guidewire 28 in a sliding fashion. The guidewire 28 and catheter 10 may thus be advanced or withdrawn independently, or the catheter 10 may be guided along a path selected with the guidewire 28.

In the illustrated embodiment, a distal portion of the shaft 14 is constructed of an inner and outer tubular body 22 and 24. The inner body 22 defines the guidewire lumen 26, while a distal portion of the inflation lumen 18 is defined by an annular space between the inner and outer tubular bodies 22 and 24. The guidewire lumen 26 extends through the inner tubular body 22 from a distal guidewire port 30 near the catheter distal end to a proximal guidewire port 32 near a proximal end of the inner body 22.

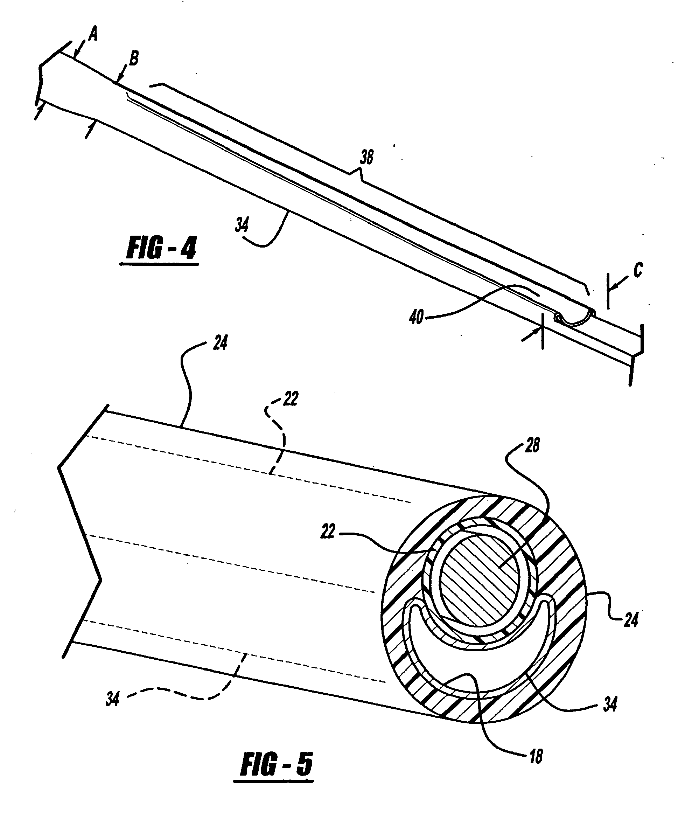

A proximal portion of the shaft 14 is constructed of a hypotube 34 component. The hypotube 34 is affixed to the hub 16, and has a proximal cylindrical tubular portion 36, an intermediate tubular portion 38 with a longitudinal indentation 40, and a distal portion 42 with an arcuate cross-section. The proximal cylindrical tubular portion 36 may of course have a taper, as indicated in Figure 4 with outer dimensions A and B, in which dimension A may be larger than dimension B for example. This taper is also indicated in Figure 8a with angle D.

The longitudinal indentation 40 is shallower in the proximal direction, and deeper in the distal direction. A transition point is 44 defined on the hypotube 34 at a distal end of the longitudinally indented intermediate tubular portion 38, and at a proximal end of the distal portion 42 of the hypotube. The longitudinal position of this transition is indicated in Figure 4 as location C. At or near this transition 44, the proximal ends of the inner and outer tubular bodies 22 and 24 are sealed to the hypotube 34. At this transition seal, the proximal end of the inner tubular body 22 is partially received within a distal portion of the longitudinal indentation 40 of the hypotube 34.

The hypotube is preferably made of metal which is selected to be biocompatible, such as for example stainless steel. Other acceptable metals may include titanium, a nickel titanium based shape memory alloy such as nitinol, etc. The hypotube shaft design of the present invention provides various advantages which will become apparent to those skilled in the art, including: a proximal shaft portion that was high column strength and high torque transmission, low profile, an integral and smooth flexibility transition near the proximal guidewire port, as well as more efficient and cost-efficient manufacturing due to relatively few component parts.

The inflation lumen 18 extends from the inflation port 20, through a proximal portion of the inflation lumen 18 defined by the hypotube, through a distal portion of the inflation lumen 18 defined by the annular space between the inner and outer bodies 22 and 24, and into the balloon.

The various portions of the hypotube component are preferably integral and unitary, and may be made with any of numerous manufacturing techniques. For example, a single piece of tubing may be ground down to form the distal extension, and then the intermediate portion may be compressed to form the indentation.

The longitudinal indentation also tends to make the hypotube intermediate portion more flexible than the hypotube proximal portion, which is preferably cylindrical. Again, a relatively smooth transition in flexibility at various points along the length of the catheter is preferable.

The integration of the hypotube and distal portion, rather than a separate hypotube and stiffening wire which are affixed together, is also preferable.

Another reason the present invention improves cost-effectiveness of manufacturing is that only one intermediate shaft seal is needed, rather than a separate seal of hypotube to polymer tube and a seal at the proximal guidewire port.

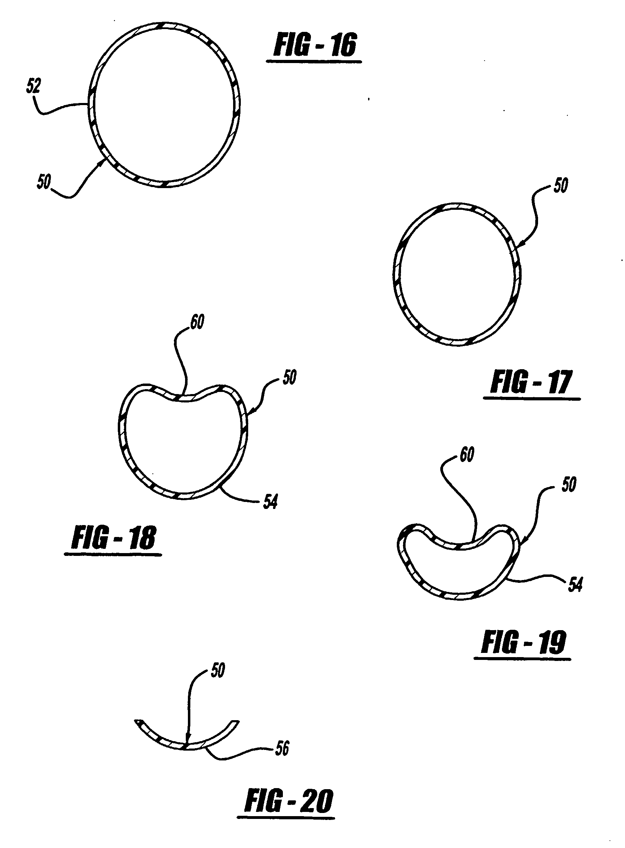

An additional arrangement of the hypotube of the present invention is shown in Figures 15-20. Hypotube 50 has a proximal tubular portion 52, an intermediate tubular portion 54 with a longitudinal indentation 60 that has a compound shape with a steeper proximal indent, and a distal portion 56 with an arcuate cross-section.

The balloon catheter and stent delivery system of the present invention may be made using various methods, many of which are known in the art, including extruding polymer tubes, injection-molding the proximal hub, and extruding a balloon parison and then blowing the parison into a balloon having the desired properties. It is also known to affix polymer components to each other by heat-sealing, or by using an adhesive such as a UV-cured adhesive.

During manufacture of the transition seal, another advantage of the present invention is that the crescent-shaped tubular portion of the hypotube at the transition will tend to hold itself open during sealing of the inner and outer bodies to the hypotube. Accordingly, a conventional removable sealing mandrel to keep the inflation lumen open during sealing is unnecessary.

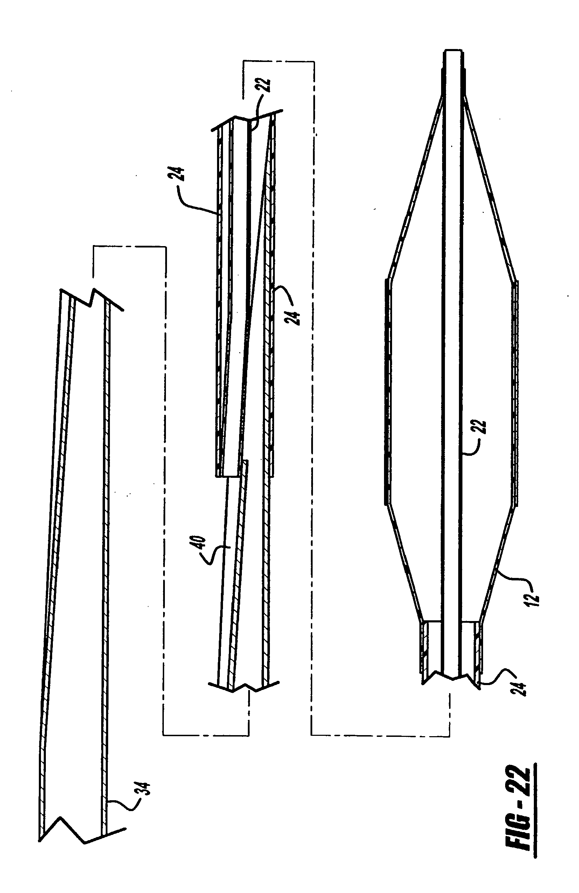

A stent of any suitable type or configuration may be provided with the catheter of the present invention, such as the well-known Palmaz-Schatz balloon expandable stent and the successful BX Velocity stent. Various kinds and types of stents are available in the market, and many different currently available stents are acceptable for use in the present invention, as well as new stents which may be developed in the future. The stent may be a cylindrical metal mesh stent having an initial crimped outer diameter, which may be forcibly expanded by the balloon to a deployed diameter. When deployed in a body passageway of a patient, the stent may be designed to preferably press radially outward to hold the passageway open. Figure 22 shows a cross-section view of a balloon catheter with a stent 46.

## Claims

1. A balloon catheter having a proximal end and a distal end for medically treating a patient, comprising:
a hypotube (34; 50) having a proximal tubular portion (36; 52), an intermediate tubular portion (38; 54) having a longitudinal indentation (40; 60), and a distal portion (42; 56);
an inner tubular body (22) having a proximal and distal end, and defining a proximal and distal guidewire port at each end respectively, and a guidewire lumen (26) extending between the guidewire ports;
an outer tubular body (24) surrounding at least a portion of the inner tubular body (22); the proximal ends of the inner and outer tubular bodies being affixed together and sealed to the hypotube (34; 50) at a point defined at or near a transition between the intermediate and distal portions of the hypotube (34; 50);
a balloon (12) affixed to the inner and outer tubular bodies at or near their distal ends;
an inflation lumen (18) extending from the hypotube proximal end, through the hypotube (34; 50) proximal and intermediate tubular portions, and through an annular space between the outer and inner tubular bodies, into an interior of the balloon;
the distal portion (42; 56) of the hypotube (34;50) extending a distance into the outer tubular body (24); providing a transition in flexibility between the tubular portions of the hypotube to the inner and outer bodies;
the balloon catheter thus having a rapid-exchange configuration.

2. The balloon catheter of Claim 1, wherein the balloon (12) has a central inflatable portion between a proximal collar and a distal collar each affixed to the catheter shaft; the balloon (12) in an initial configuration being deflated, pleated and wrapped around the catheter shaft.

3. The balloon catheter of Claim 1, wherein the hypotube (34; 50) is integral and unitary.

4. The balloon catheter of Claim 1, wherein the distal portion (42; 56) of the hypotube has an arcuate cross-section.

5. The balloon catheter of Claim 1, wherein the longitudinal indentation (40; 60) is shallower in a proximal direction, and deeper in a distal direction.

6. The balloon catheter of Claim 1, wherein the proximal portion (36; 52) of the hypotube is cylindrical.

7. The balloon catheter of Claim 6, further comprising a tapering portion between the proximal cylindrical portion (36; 52) and the indented intermediate portion (38; 54).

8. The balloon catheter of Claim 1 further comprising a stent crimped around the balloon (12).

9. The balloon catheter of Claim 1, wherein the proximal ends of the inner and outer tubular bodies are sealed to the hypotube (34; 50) with a single seal.

10. The balloon catheter of Claim 1, wherein the hypotube (34; 50) is made of stainless steel.

## Patentansprüche

1. Ballonkatheter mit einem proximalen Ende und einem distalen Ende zur medizinischen Behandlung eines Patienten, wobei der Ballonkatheter Folgendes umfasst:
eine Hyporöhre (34; 50) mit einem proximalen röhrenförmigen Abschnitt (36; 52), einem mittleren röhrenförmigen Abschnitt (38; 54) mit einer länglichen Einbuchtung (40; 60) und einem distalen Abschnitt (42; 56);
ein innerer röhrenförmiger Körper (22) mit einem proximalen und einem distalen Ende, der einen proximalen und einen distalen Führungsdrahtanschluss an dem jeweiligen Ende definiert und bei der sich ein Führungsdrahtlumen (26) zwischen den Führungsdrahtanschlüssen erstreckt;
ein äußerer röhrenförmiger Körper (24), der wenigstens einen Abschnitt des inneren röhrenförmigen Körpers (22) umgibt, wobei die proximalen Enden der inneren und der äußeren röhrenförmigen Körper aneinander befestigt sind und an der Hyporöhre (34; 50) an einem Punkt abgedichtet ist, der an oder nahe eines Überganges zwischen dem mittleren und dem distalen Abschnitt der Hyporöhre (34; 50) definiert ist;
ein Ballon (12), der an dem inneren und dem äußeren röhrenförmigen Körper an oder nahe deren distalen Enden befestigt ist;
ein Aufblaslumen (18), das sich von dem proximalen Ende der Hyporöhre, durch den proximalen und mittleren röhrenförmigen Abschnitt der Hyporöhre (34; 50) und durch einen ringförmigen Raum zwischen dem äußeren und dem inneren röhrenförmigen Körper in einen Innenraum des Ballons erstreckt;
wobei sich der distale Abschnitt (42; 56) der Hyporöhre (34; 50) über eine Strecke in den äußeren röhrenförmigen Körper (24) erstreckt und für einen Übergang in der Flexibilität zwischen den röhrenförmigen Abschnitten der Hyporöhre zu dem inneren und dem äußeren Körper sorgt;
wobei der Ballonkatheter auf diese Weise eine Schnellaustauschkonfiguration aufweist.

2. Ballonkatheter nach Anspruch 1, bei welchem der Ballon (12) einen zentralen Aufblasabschnitt zwischen einer proximalen Manschette und einer distalen Manschette, die jeweils an dem Katheterschaft befestigt sind, aufweist; und der Ballon (12) entleert, gefaltet und um den Katheterschaft gewickelt ist.

3. Ballonkatheter nach Anspruch 1, bei welchem die Hyopröhre (34; 50) integral und einheitlich ausgebildet ist.

4. Ballonkatheter nach Anspruch 1, wobei der distale Abschnitt (42; 56) der Hyporöhre einen bogenförmigen Querschnitt aufweist.

5. Ballonkatheter nach Anspruch 1, bei welchem die längliche Einbuchtung (40; 60) in einer proximalen Richtung flacher ist und in einer distalen Richtung tiefer ist.

6. Ballonkatheter nach Anspruch 1, bei welchem der proximale Abschnitt (36; 52) der Hyporöhre zylindrisch ist.

7. Ballonkatheter nach Anspruch 6, der weiterhin einen sich verjüngenden Abschnitt zwischen dem proximalen zylindrischen Abschnitt (36; 52) und dem eingebuchteten mittleren Abschnitt (38; 54) umfasst.

8. Ballonkatheter nach Anspruch 1, der weiterhin einen Stent aufweist, der um den Ballon (12) gequetscht ist.

9. Ballonkatheter nach Anspruch 1, bei dem die proximalen Enden des inneren und des äußeren röhrenförmigen Körpers an der Hyporöhre (34; 50) mit einer einzelnen Dichtung abgedichtet sind.

10. Ballonkatheter nach Anspruch 1, bei welchem die Hyporöhre (34; 50) aus einem Edelstahl hergestellt ist.

## Revendications

1. Cathéter à ballonnet présentant une extrémité proximale et une extrémité distale destiné à traiter médicalement un patient, comprenant :
un hypotube (34 ; 50) présentant une partie tubulaire proximale (36 ; 52), une partie tubulaire intermédiaire (38 ; 54) présentant une indentation longitudinale (90 ; 60), et une partie distale (42 ; 56) ;
un corps tubulaire interne (22) présentant une extrémité proximale et une extrémité distale, et définissant des orifices de fil-guide proximal et distal à chaque extrémité respectivement, et une lumière de fil-guide (26) s'étendant entre les orifices de fil-guide ;
un corps tubulaire externe (24) entourant au moins une partie du corps tubulaire interne (22) ; les extrémités proximales des corps tubulaires interne et externe étant fixées les unes aux autres et scellées à l'hypotube (34 ; 50) en un point défini au niveau ou à proximité d'une transition entre les parties intermédiaire et distale de l'hypotube (34 ; 50) ;
un ballonnet (12) fixé aux corps tubulaires interne et externe au niveau ou à proximité de leurs extrémités distales ;
une lumière de gonflement (18) s'étendant depuis l'extrémité proximale de l'hypotube, à travers les parties tubulaires proximale et intermédiaire de l'hypotube (34 ; 50), et à travers un espace annulaire entre les corps tubulaires externe et interne, à l'intérieur du ballonnet ;
la partie distale (42 ; 56) de l'hypotube (34 ; 50) s'étendant le long d'une distance à l'intérieur du corps tubulaire externe (24) ; fournissant une transition de flexibilité entre les parties tubulaires de l'hypotube vers les corps interne et externe ;
le cathéter à ballonnet présentant ainsi une configuration d'échange rapide.

2. Cathéter à ballonnet selon la revendication 1, dans lequel le ballonnet (12) présente une partie gonflable centrale entre un collier proximal et un collier distal fixés chacun à la tige du cathéter ; le ballonnet (12) dans une configuration initiale étant dégonflé, plissé et enveloppé autour de la tige du cathéter.

3. Cathéter à ballonnet selon la revendication 1, dans lequel l'hypotube (34 ; 50) est d'une pièce et unitaire.

4. Cathéter à ballonnet selon la revendication 1, dans lequel la partie distale (42 ; 56) de l'hypotube présente une section transversale arquée.

5. Cathéter à ballonnet selon la revendication 1, dans lequel l'indentation longitudinale (40 ; 60) est moins profonde dans une direction proximale, et plus profonde dans une direction distale.

6. Cathéter à ballonnet selon la revendication 1, dans lequel la partie proximale (36 ; 52) de l'hypotube est cylindrique.

7. Cathéter à ballonnet selon la revendication 6, comprenant en outre une partie biseautée entre la partie cylindrique proximale (36 ; 52) et la partie intermédiaire dentée (38 ; 54).

8. Cathéter à ballonnet selon la revendication 1 comprenant en outre une endoprothèse vasculaire sertie autour du ballonnet (12).

9. Cathéter à ballonnet selon la revendication 1, dans lequel les extrémités proximales des corps tubulaires interne et externe sont scellées à l'hypotube (34 ; 50) avec un seul joint.

10. Cathéter à ballonnet selon la revendication 1, dans lequel le tue hypodermique (39 ; 50) est fabriqué en acier inoxydable.
